# EUROPEAN PATENT SPECIFICATION

(11) **EP 4 498 918 B1**
(45) Date of publication and mention of the grant of the patent: **28.05.2025**
(21) Application number: 23726849.5
(22) Date of filing: 20.04.2023
(51) Int. Cl.: H05G 1/10, A61B 6/00

(54) **MEDICAL X-RAY IMAGING DEVICE AND POWER SUPPLY SYSTEM THEREFOR**
MEDIZINISCHE RÖNTGENBILDGEBUNGSVORRICHTUNG UND STROMVERSORGUNGSSYSTEM DAFÜR
DISPOSITIF D'IMAGERIE MÉDICALE À RAYONS X ET SYSTÈME D'ALIMENTATION ÉLECTRIQUE ASSOCIÉ

(30) Priority: 10.05.2022 CN 202221198333 U
(43) Date of publication of application: 05.02.2025
(73) Proprietor: Siemens Shanghai Medical Equipment Ltd., Shanghai 201318 (CN)
(72) Inventor: CHEN, Zhida, Shanghai 200051 (CN)
(74) Representative: Horn Kleimann Waitzhofer Schmid-Dreyer Patent- und Rechtsanwälte PartG mbB
(86) International application number: PCT/CN2023/089571
(87) International publication number: WO 2023/216835

(56) References cited:
- DE-A1- 102010 042 565
- JP-A- H0 856 939
- US-A- 5 226 064

## Description

### TECHNICAL FIELD

The present application relates to a power supply system, and particularly relates to a power supply system for a medical X-ray imaging device and the medical X-ray imaging device including the power supply system.

### BACKGROUND

At present, movable products of a medical X-ray imaging device, such as a movable C-shaped arm X-ray machine, have been developed. The movable products can easily move between different operating rooms of a hospital. In this process, it is necessary to disconnect and reconnect a power supply as well as close and restart the device. This process takes a long time, and the device cannot be used in emergency.

DE 10 2010042565 A1 discloses a device for supplying electrical power to an image-generating medical device. The device comprises a charger connected with an electrical power supply for generating at least one operation DC voltage from an AC voltage of the electrical power supply. An energy storage device is connected to the charger and provided with a lithium ions accumulator. The an input side of the energy storage device is provided with the operation DC voltage as charging voltage. An output side of an energy storage device provides directly the operation DC voltage for performing operation of the imaging medical device.

US 5226064 A discloses that, in a CT (computerized tomographic) scanning apparatus, a biological body under medical examination is scanned to acquire image data on the scanned body. The CT scanning apparatus comprises: an AC/DC converting unit for converting AC power supplied from a commercial power source into first DC power; a peak power consumption unit for consuming peak power during a scanning operation; and a secondary battery unit rechargeable by the first DC power supplied from the AC/DC converting unit and capable of supplying second DC power to the peak power consumption unit during at least the scanning operation.

JP H08-56939 A discloses a CT scanner. To cope with a peak power even when the power capacity of an engine generator is reduced, a secondary battery capable of charging a total power required for one time of scan or plural times of scan is provided in an X-ray high voltage device. The total power required for the scan is charged into the secondary battery in a period while the scan is interrupted, and the power is supplied from the secondary battery to the first unit of an X-ray tube, etc., when the scan is performed.

### SUMMARY

One objective of the present application is to provide a power supply system for a medical X-ray imaging device. The power supply system can reduce the time for reconnecting the medical X-ray imaging device to a power supply to recover to a normal working state.

Another objective of the present application is to provide the medical X-ray imaging device. The power supply system for the medical X-ray imaging device can reduce the time for reconnecting the medical X-ray imaging device to the power supply to recover to the normal working state.

The present application provides a power supply system for a medical X-ray imaging device. The power supply system includes a power connector, a first power supply connector, a battery module and a second power supply connector. The power connector is configured to be connected to a power grid. The first power supply connector is connected to the power connector. The first power supply connector is configured to be connected to a first-class electrical component of the medical X-ray imaging device. The battery module is connected to the power connector. The battery module is provided with a storage battery. The battery module is configured to acquire electric energy from the power grid through the power connector to charge the storage battery. The second power supply connector is connected to the battery module. The second power supply connector is configured to be connected to a second-class electrical component of the medical X-ray imaging device. The battery module is configured to connect any one of the power connector and the storage battery to the second power supply connector in a switchable mode.

According to the power supply system for a medical X-ray imaging device, the second-class electrical component can obtain electric energy from the storage battery to maintain operating in a case that the power connector is in a power-off state, so that when the power connector is reconnected to the power grid, the second-class electrical component does not need to be restarted, and only the electrical component with the restarting time less than a preset time threshold, namely the first-class electrical component, needs to be restarted. Therefore, the power supply system can effectively reduce the time for reconnecting the medical X-ray imaging device to the power supply to recover to the normal working state.

The battery module includes a first switch. The first switch is controlled by the power connector. A contact end of the first switch is connected to the power connector, the storage battery and the second power supply connector. The first switch is configured to connect the power connector to the second power supply connector in a case that the power connector is in a power-on state; and the first switch is configured to connect the storage battery to the second power supply connector in a case that the power connector is in a power-off state. By arranging the first switch, connection of the power connector, the storage battery and the second power supply connector can be automatically switched according to a condition whether the power connector is powered on or not, and therefore the convenience in use can be improved.

In another exemplary embodiment of the power supply system for a medical X-ray imaging device, the first switch is a relay.

In yet another exemplary embodiment of the power supply system for a medical X-ray imaging device, the battery module includes a first AC/DC converter and a first DC/AC converter. The first AC/DC converter is connected between the power connector and the storage battery. The first DC/AC converter is connected between the storage battery and the first switch. Therefore, the electric energy can be switched between alternating current and direct current according to the requirements of the storage battery and AC-powered electrical components.

In still yet another exemplary embodiment of the power supply system for a medical X-ray imaging device, the power supply system further includes a second AC/DC converter. The second AC/DC converter is connected to the first power supply connector and is configured to be connected to a DC-powered first-class electrical component. Therefore, the electric energy is conveniently supplied to the DC-powered first-class electrical component.

In still yet another exemplary embodiment of the power supply system for a medical X-ray imaging device, the power supply system further includes a third AC/DC converter. The third AC/DC converter is connected to the second power supply connector and configured to be connected to a DC-powered second-class electrical component. Therefore, the electric energy is conveniently supplied to the DC-powered second-class electrical component.

In still yet another exemplary embodiment of the power supply system for a medical X-ray imaging device, the power supply system includes two second power supply connectors; one second power supply connector is configured to be connected to an AC-powered second-class electrical component, and the other second power supply connector is configured to be connected to the DC-powered second-class electrical component. The contact end of the first switch is provided with a first contact set and a second contact set. The first contact set is connected to the second power supply connector which is configured to be connected to the AC-powered second-class electrical component, and the second contact set is connected to the second power supply connector which is configured to be connected to the DC-powered second-class electrical component. The battery module further includes a first AC/DC converter, a first DC/AC converter and a DC/DC converter. The first AC/DC converter is connected between the power connector and the storage battery. The first DC/AC converter is connected between the storage battery and the first contact set. The DC/DC converter is connected between the storage battery and the second contact set. Therefore, the electric energy can be switched between alternating current and direct current according to the requirements of the storage battery and the electrical components.

In still yet another exemplary embodiment of the power supply system for a medical X-ray imaging device, the power supply system further includes a second AC/DC converter. The second AC/DC converter is connected to the first power supply connector and configured to be connected to the DC-powered first-class electrical component. The second contact set is connected to the power connector through the second AC/DC converter and the first power supply connector. Therefore, a circuit can be simplified, and the stability of the power supply system can be improved.

In still yet another exemplary embodiment of the power supply system for a medical X-ray imaging device, the battery module further includes a battery management system. The battery management system is configured to detect a residual electric quantity of the storage battery and transmit an electric quantity signal. The power supply system further includes a prompt module. The prompt module is connected to the battery management system and is configured to display patterns or make a sound according to the electric quantity signal. Therefore, a user can conveniently know the residual electric quantity of the storage battery.

In still yet another exemplary embodiment of the power supply system for a medical X-ray imaging device, the battery module is an uninterruptible power supply. The power supply system further includes a second switch and a third power supply connector. The third power supply connector is configured to be connected to a second-class electrical component needing limitation on operating power in power off. The second switch is controlled by the power connector. A contact end of the second switch is connected to the power connector, the second power supply connectors and the third power supply connector. The second switch is configured to connect the power connector to the third power supply connector in a case that the power connector is in the power-on state, and the second switch is configured to connect the second power supply connectors to the third power supply connector in a case that the power connector is in the power-off state. By arranging the uninterruptible power supply, the circuit can be simplified, and the stability of the power supply system can be improved. By arranging the second switch and the third power supply connector, a situation that the maximum output power of the uninterruptible power supply limits the operating power of the electrical components in a case that the power connector is in the power-on state can be avoided.

In still yet another exemplary embodiment of the power supply system for a medical X-ray imaging device, the second switch is a relay.

In still yet another exemplary embodiment of the power supply system for a medical X-ray imaging device, the power supply system further includes a monitoring module. The monitoring module is configured to detect an on-off state of the contact end of the second switch and transmit an operating power limiting signal in a case of detecting that the second switch connects the second power supply connectors to the third power supply connector. A control system for a medical X-ray imaging device is configured to control the second-class electrical component needing limitation on operating power in power off to only implement a function that the power is less than a set threshold according to the operating power limiting signal. Therefore, a situation that the device is damaged because of forcibly operating a function that the power is greater than the maximum output power of the uninterruptible power supply in a case that the power connector is in the power-off state can be prevented.

In still yet another exemplary embodiment of the power supply system for a medical X-ray imaging device, the power supply system further includes an AC/AC converter. The AC/AC converter is connected between the power connector and the first power supply connector and between the power connector and the battery module.

The present application further provides a medical X-ray imaging device. The medical X-ray imaging device includes the abovementioned power supply system. The power supply system for the medical X-ray imaging device can effectively reduce the time for reconnecting the medical X-ray imaging device to the power supply to recover to the normal working state.

In another exemplary embodiment of the medical X-ray imaging device, the electrical components of the medical X-ray imaging device are divided into the first-class electrical component and the second-class electrical component according to the time for restarting; the restarting time of the first-class electrical component is less than a preset time threshold; and the restarting time of the second-class electrical component is greater than or equal to the preset time threshold.

In yet another exemplary embodiment of the medical X-ray imaging device, the medical X-ray imaging device is a movable C-shaped arm X-ray machine; and the second-class electrical component of the movable C-shaped arm X-ray machine includes an X-ray generator, a computer, a collimator and a micro-control unit.

### BRIEF DESCRIPTION OF THE DRAWINGS

The following drawings are only used to schematically explain the present embodiments, and do not limit the scope of the present embodiments.
FIG. 1 is a schematic circuit diagram of a power supply system for a medical X-ray imaging device of an exemplary embodiment.
FIG. 2 is a schematic circuit diagram of a power supply system for a medical X-ray imaging device of another exemplary embodiment.
FIG. 3 is a schematic circuit diagram of a power supply system for a medical X-ray imaging device of yet another exemplary embodiment.

### Reference numerals

10, power connector
21, first power supply connector
22, second power supply connector
23, third power supply connector
30, battery module
31, storage battery
32, first switch
33, first AC/DC converter
34, first DC/AC converter
35, DC/DC converter
36, battery management system
42, second AC/DC converter
43, third AC/DC converter
44, AC/AC converter
50, prompt module
60, second switch
70, monitoring module
81, AC-powered first-class electrical component
82, DC-powered first-class electrical component
91, AC-powered second-class electrical component
911, AC-powered second-class electrical component needing limitation on operating power in power off
912, AC-powered second-class electrical component not needing limitation on operating power in power off
92, DC-powered second-class electrical component

### DETAILED DESCRIPTION

In order to have a clearer understanding of the technical characteristics, objectives, and effects of the present application, the specific embodiments of the present application are explained with reference to the accompanying drawings. The same reference numerals in each figure represent components with the same structure or similar structure and the same function.

In the present applicatipon, "exemplary" means "serving as an instance, example, or explanation", and any illustration or embodiment described as "exemplary" in the present application cannot be interpreted as a more preferred or advantageous technical solution.

In the present application, "first", "second", etc. are not used to indicate their importance or order, but only to indicate their differences and facilitate the description of the document.

To make the drawings concise, only the parts related to the present application are schematically represented in each figure, and they do not represent the actual structure of a product.

In the present application, "AC" represents alternating current, and "DC" represents direct current. An AC/DC converter refers to an element that converts AC into DC. A DC/AC converter refers to an element that converts DC into AC. An AC/AC converter refers to an element that converts AC of one form into AC of another form. A DC/DC converter refers to an element that converts DC of one form into DC of another form.

The present application provides a power supply system for a medical X-ray imaging device. Electrical components of the medical X-ray imaging device are divided into a first-class electrical component and a second-class electrical component according to the time for restarting; the restarting time of the first-class electrical component is less than a preset time threshold; and the restarting time of the second-class electrical component is greater than or equal to the preset time threshold. The specific numerical value of the time threshold can be flexibly adjusted according to actual needs. The medical X-ray imaging device can be, for example, a movable C-shaped arm X-ray machine. The second-class electrical component of the movable C-shaped arm X-ray machine includes an X-ray generator, a computer, a collimator and a micro-control unit.

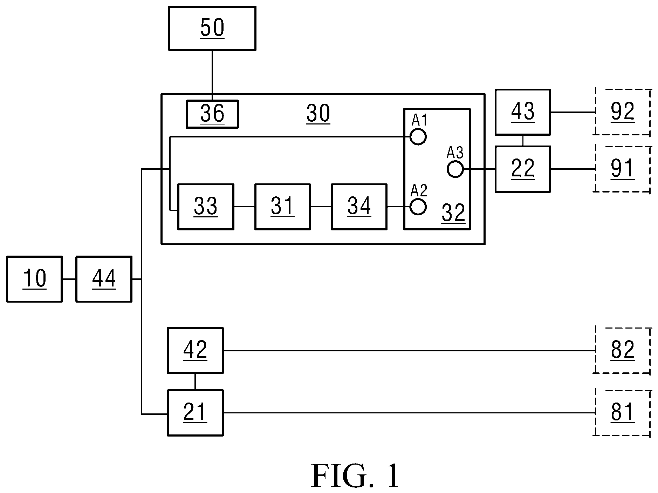

FIG. 1 is a schematic circuit diagram of a power supply system for a medical X-ray imaging device of an exemplary embodiment. As shown in FIG. 1, the power supply system for a medical X-ray imaging device includes a power connector 10, a first power supply connector 21, a battery module 30 and a second power supply connector 22.

The power connector 10 is configured to be connected to a power grid. The first power supply connector 21 is connected to the power connector 10. The first power supply connector 21 is configured to be connected to a first-class electrical component (including an AC-powered first-class electrical component 81 and a DC-powered first-class electrical component 82) of the medical X-ray imaging device. The battery module 30 is connected to the power connector 10. The battery module 30 is provided with a storage battery 31. The battery module 30 is configured to acquire electric energy from the power grid through the power connector 10 to charge the storage battery 31. The second power supply connector 22 is connected to the battery module 30. The second power supply connector 22 is configured to be connected to a second-class electrical component (including an AC-powered second-class electrical component 91 and a DC-powered second-class electrical component 92) of the medical X-ray imaging device. The battery module 30 is configured to connect any one of the power connector 10 and the storage battery 31 to the second power supply connector 22 in a switchable mode. For example, switching is achieved in a manual control or automatic control mode. In this exemplary embodiment, the battery module 30 achieves automatic switching through a switch, but it is not limited to this.

The working process of the power supply system is illustrated as follows.

In a case that the power connector 10 is in a power-on state (namely, the power connector 10 is connected to the power grid), the first-class electrical component (including the AC-powered first-class electrical component 81 and the DC-powered first-class electrical component 82) can obtain electric energy from the power grid through the power connector 10. Meanwhile, the battery module 30 is switched to connect the power connector 10 to the second power supply connector 22, and the second-class electrical component (including the AC-powered second-class electrical component 91 and the DC-powered second-class electrical component 92) can obtain electric energy from the power grid through the power connector 10. Meanwhile, the battery module 30 can obtain the electric energy from the power grid through the power connector 10 to charge the storage battery 31.

In a case that the power connector 10 is in a power-off state (namely, the power connector 10 is not connected to the power grid), the first-class electrical component (including the AC-powered first-class electrical component 81 and the DC-powered first-class electrical component 82) is powered off. Meanwhile, the battery module 30 is switched to connect the storage battery 31 to the second power supply connector 22, and the second-class electrical component (including the AC-powered second-class electrical component 91 and the DC-powered second-class electrical component 92) can obtain electric energy from the storage battery 31 to maintain operating.

When the power connector 10 is reconnected to the power grid, the first-class electrical component (including the AC-powered first-class electrical component 81 and the DC-powered first-class electrical component 82) can obtain electric energy from the power grid through the power connector 10 to be restarted. Meanwhile, the battery module 30 is switched to connect the power connector 10 to the second power supply connector 22, and the second-class electrical component (including the AC-powered second-class electrical component 91 and the DC-powered second-class electrical component 92) can obtain electric energy from the power grid through the power connector 10. Meanwhile, the battery module 30 can obtain electric energy from the power grid through the power connector 10 to charge the storage battery 31 again.

As the second-class electrical component can obtain electric energy from the storage battery 31 to maintain operating in a case that the power connector 10 is in the power-off state, when the power connector 10 is reconnected to the power grid, the second-class electrical component does not need to be restarted, and only the electrical component with the restarting time less than a preset time threshold, namely the first-class electrical component, needs to be restarted. Therefore, the power supply system can effectively reduce the time for reconnecting the medical X-ray imaging device to the power supply to recover to the normal working state.

As shown in FIG. 1, in this exemplary embodiment, the battery module 30 includes a first switch 32 (only a contact end of the first switch is drawn in FIG. 1). The first switch 32 is controlled by the power connector 10, for example, the first switch is a relay, but it is not limited to this. The contact end of the first switch 32 is connected to the power connector 10, the storage battery 31 and the second power supply connector 22, specifically, a contact A1 is connected to the power connector 10, a contact A2 is connected to the storage battery 31, and a contact A3 is connected to the second power supply connector 22. The first switch 32 is configured to connect the power connector 10 to the second power supply connector 22 in a case that the power connector 10 is in a power-on state (namely, the contact A1 makes contact with the contact A3, and the contact A2 is disconnected from the contact A3), and the first switch 32 is configured to connect the storage battery 31 to the second power supply connector 22 in a case that the power connector 10 is in a power-off state (namely, the contact A2 makes contact with the contact A3, and the contact A1 is disconnected from the contact A3). By arranging the first switch, connection of the power connector 10, the storage battery 31 and the second power supply connector 22 can be automatically switched according to a condition whether the power connector 10 is powered on or not, and therefore the convenience in use can be improved.

As shown in FIG. 1, in this exemplary embodiment, the battery module 30 further includes a first AC/DC converter 33 and a first DC/AC converter 34. The first AC/DC converter 33 is connected between the power connector 10 and the storage battery 31. The first DC/AC converter 34 is connected between the storage battery 31 and the contact A2 of the first switch 32. As current in the power grid is alternating current, the storage battery 31 is charged with direct current, and current in some electrical components is alternating current; and the first AC/DC converter 33 and the first DC/AC converter 34 are arranged to switch electric energy between alternating current and direct current according to the requirements of the storage battery 31 and AC-powered electrical components.

As shown in FIG. 1, in this exemplary embodiment, the power supply system further includes a second AC/DC converter 42 and a third AC/DC converter 43. The second AC/DC converter 42 is connected to the first power supply connector 21 and configured to be connected to the DC-powered first-class electrical component 82. The AC-powered first-class electrical component 81 is directly connected to the first power supply connector 21. The third AC/DC converter 43 is connected to the second power supply connector 22 and configured to be connected to the DC-powered second-class electrical component 92. The AC-powered second-class electrical component 91 is directly connected to the second power supply connector 22. Electric energy can be conveniently supplied to the DC-powered electrical components through the second AC/DC converter 42 and the third AC/DC converter 43.

As shown in FIG. 1, in this exemplary embodiment, the power supply system further includes an AC/AC converter 44. The AC/AC converter 44 is connected between the power connector 10 and the first power supply connector 21 and between the power connector 10 and the battery module 30. Therefore, the alternating current provided by the power grid is converted into alternating current meeting the requirements of the medical X-ray imaging device.

As shown in FIG. 1, in this exemplary embodiment, the battery module 30 further includes a battery management system 36. The battery management system 36 is configured to detect a residual electric quantity of the storage battery 31 and transmit an electric quantity signal. The power supply system further includes a prompt module 50. The prompt module 50 is connected to the battery management system 36 and is configured to display patterns or make a sound according to the electric quantity signal. The prompt module 50 is a display for example, and is configured to display different patterns to represent different residual electric quantities of the storage battery 31. The prompt module 50 is also a buzzer for example, and is configured to make a sound to remind a user when the residual electric quantity of the storage battery 31 is less than a threshold.

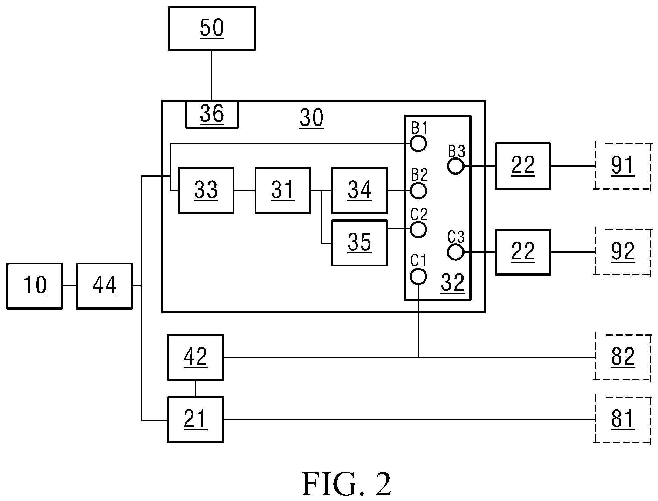

FIG. 2 is a schematic circuit diagram of a power supply system for a medical X-ray imaging device of another exemplary embodiment. As shown in FIG. 2, the power supply system for a medical X-ray imaging device includes a power connector 10, a first power supply connector 21, a battery module 30 and two second power supply connectors 22.

The power connector 10 is configured to be connected to a power grid. The first power supply connector 21 is connected to the power connector 10. The first power supply connector 21 is configured to be connected to a first-class electrical component (including an AC-powered first-class electrical component 81 and a DC-powered first-class electrical component 82) of the medical X-ray imaging device. The battery module 30 is connected to the power connector 10. The battery module 30 is provided with a storage battery 31. The battery module 30 is configured to acquire electric energy from the power grid through the power connector 10 to charge the storage battery 31. The second power supply connector 22 is connected to the battery module 30. The second power supply connectors 22 are configured to be connected to a second-class electrical component of the medical X-ray imaging device; one second power supply connector 22 is configured to be connected to an AC-powered second-class electrical component 91, and the other second power supply connector 22 is configured to be connected to a DC-powered second-class electrical component 92. The battery module 30 is configured to connect any one of the power connector 10 and the storage battery 31 to the second power supply connectors 22 in a switchable mode.

Specifically, as shown in FIG. 2, the battery module 30 includes a first switch 32 (only a contact end of the first switch is drawn in FIG. 2). The first switch 32 is controlled by the power connector 10, for example, the first switch is a relay, but it is not limited to this. The contact end of the first switch 32 is provided with a first contact set (i.e., contacts B1, B2 and B3) and a second contact set (i.e., contacts C1, C2 and C3). In the first contact set, the contact B1 is connected to the power connector 10, the contact B2 is connected to the storage battery 31, and the contact B3 is connected to the second power supply connector 22 which is configured to be connected to the AC-powered second-class electrical component 91. In the second contact set, the contact C1 is connected to the power connector 10, the contact C2 is connected to the storage battery 31, and the contact C3 is connected to the second power supply connector 22 which is configured to be connected to the DC-powered second-class electrical component 92.

As shown in FIG. 2, in this exemplary embodiment, the battery module 30 further includes a first AC/DC converter 33, a first DC/AC converter 34 and a DC/DC converter 35. The first AC/DC converter 33 is connected between the power connector 10 and the storage battery 31. The first DC/AC converter 34 is connected between the storage battery 31 and the contact B2 of the first contact set. The DC/DC converter 35 is connected between the storage battery 31 and the contact C2 of the second contact set.

The first switch 32 is configured to connect the power connector 10 to the second power supply connectors 22 (namely, the contact B1 makes contact with the contact B3, and the contact C1 makes contact with the contact C3) in a case that the power connector 10 is in a power-on state; and the first switch 32 is configured to connect the storage battery 31 to the second power supply connectors 22 (namely, the contact B2 makes contact with the contact B3, and the contact C2 makes contact with the contact C3) in a case that the power connector 10 is in a power-off state. By arranging the first switch, connection of the power connector 10, the storage battery 31 and the second power supply connector 22 can be automatically switched according to a condition whether the power connector 10 is powered on or not, and therefore the convenience in use can be improved.

As shown in FIG. 2, in this exemplary embodiment, the power supply system further includes a second AC/DC converter 42. The second AC/DC converter 42 is connected to the first power supply connector 21 and configured to be connected to the DC-powered first-class electrical component 82. The AC-powered first-class electrical component 81 is directly connected to the first power supply connector 21. The contact C1 of the second contact set is connected to the power connector 10 through the second AC/DC converter 42 and the first power supply connector 21. Electric energy can be conveniently supplied to the DC-powered electrical components through the second AC/DC converter 42.

As shown in FIG. 2, in this exemplary embodiment, the power supply system further includes an AC/AC converter 44. The AC/AC converter 44 is connected between the power connector 10 and the first power supply connector 21 and between the power connector 10 and the battery module 30. Therefore, the alternating current provided by the power grid is converted into alternating current meeting the requirements of the medical X-ray imaging device.

As shown in FIG. 2, in this exemplary embodiment, the battery module 30 further includes a battery management system 36. The battery management system 36 is configured to detect a residual electric quantity of the storage battery 31 and transmit an electric quantity signal. The power supply system further includes a prompt module 50. The prompt module 50 is connected to the battery management system 36 and is configured to display patterns or make a sound according to the electric quantity signal. The prompt module 50 is a display for example, and is configured to display different patterns to represent different residual electric quantities of the storage battery 31. The prompt module 50 is also a buzzer for example, and is configured to make a sound to remind a user when the residual electric quantity of the storage battery 31 is less than a threshold.

The schematic explanation of the working process of the power supply system is the same as that of the working process of the power supply system shown in FIG. 1, so it is not repeated herein.

As the second-class electrical component can obtain electric energy from the storage battery 31 to maintain operating in a case that the power connector 10 is in the power-off state, when the power connector 10 is reconnected to the power grid, the second-class electrical component does not need to be restarted, and only the electrical component with the restarting time less than a preset time threshold, namely the first-class electrical component, needs to be restarted. Therefore, the power supply system can effectively reduce the time for reconnecting the medical X-ray imaging device to the power supply to recover to the normal working state.

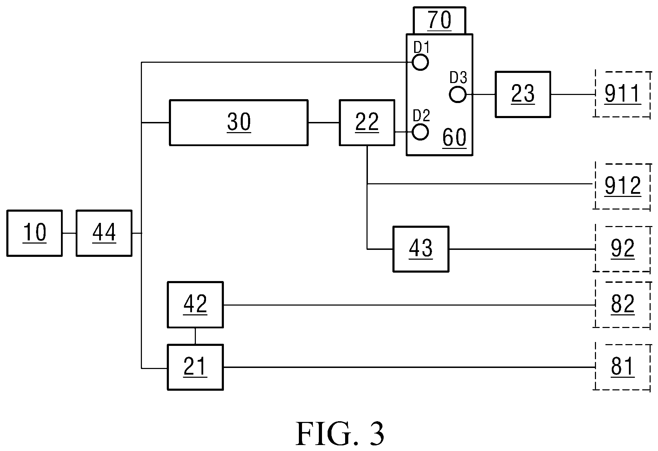

FIG. 3 is a schematic circuit diagram of a power supply system for a medical X-ray imaging device of yet another exemplary embodiment. The power supply system shown in FIG. 3 is the same as or similar to the power supply system shown in FIG. 1, and is not repeated herein, and the difference is as follows.

As shown in FIG. 3, in this exemplary embodiment, the battery module 30 is an uninterruptible power supply. A second-class electrical component of the medical X-ray imaging device can be divided into a second-class electrical component needing limitation on operating power in power off and a second-class electrical component not needing limitation on operating power in power off according to, for example, the maximum operating power in operation. The maximum operating power of the second-class electrical component needing limitation on operating power in power off is greater than the maximum output power of the uninterruptible power supply, for example, the second-class electrical component is an X-ray generator, the exposure power of the second-class electrical component is greater than the maximum output power of the uninterruptible power supply, and the power for maintaining the power-on state is less than or equal to the maximum output power of the uninterruptible power supply. The maximum operating power of the second-class electrical component not needing limitation on operating power in power off is less than or equal to the maximum output power of the uninterruptible power supply.

As shown in FIG. 3, in this exemplary embodiment, the power supply system further includes a second switch 60 (only a contact end of the second switch is drawn in FIG. 3) and a third power supply connector 23. The third power supply connector 23 is configured to be connected to an AC-powered second-class electrical component 911 needing limitation on operating power in power off in second-class electrical component. An AC-powered second-class electrical component 912 not needing limitation on operating power in power off is directly connected to the second power supply connectors 22. The second switch 60 is controlled by the power connector 10, for example, the second switch is a relay, but it is not limited to this. In the contact end of the second switch 60, a contact D1 is connected to the power connector 10, a contact D2 is connected to the second power supply connectors 22, and a contact D3 is connected to the third power supply connector 23. The second switch 60 is configured to connect the power connector 10 to the third power supply connector 23 in a case that the power connector 10 is in the power-on state (namely the contact D1 makes contact with the contact D3). The second switch 60 is configured to connect the second power supply connectors 22 to the third power supply connector 23 in a case that the power connector 10 is in the power-off state (namely the contact D2 makes contact with the contact D3). In a case that the second power supply connector 22 is connected to the third power supply connector 23, the AC-powered second-class electrical component 911 needing limitation on operating power can only start a function that the operating power is less than or equal to the maximum output power of the uninterruptible power supply. For example, the X-ray generator can only maintain the power-on state and cannot be exposed. By arranging the uninterruptible power supply, the circuit can be simplified, and the stability of the power supply system can be improved. By arranging the second switch 60 and the third power supply connector 23, a situation that the maximum output power of the uninterruptible power supply limits the operating power of the electrical components in a case that the power connector 10 is in the power-on state can be avoided.

As shown in FIG. 3, in this exemplary embodiment, the power supply system further includes a monitoring module 70. The monitoring module 70 is configured to detect an on-off state of the contact end of the second switch 60 and transmit an operating power limiting signal in a case of detecting that the second switch 60 connects the second power supply connectors 22 to the third power supply connector 23. A control system for a medical X-ray imaging device is configured to control the second-class electrical component needing limitation on operating power in power off to only implement a function that the power is less than a set threshold (the maximum output power of the uninterruptible power supply is not exceeded) according to the operating power limiting signal. For example, the X-ray generator can only maintain the power-on state and cannot be exposed. Therefore, a situation that the device is damaged because of forcibly operating a function that the power is greater than the maximum output power of the uninterruptible power supply in a case that the power connector 10 is in the power-off state can be prevented.

The present utility model further provides a medical X-ray imaging device. In one exemplary embodiment, the medical X-ray imaging device includes any one of the abovementioned power supply systems. The electrical components of the medical X-ray imaging device are divided into the first-class electrical component and the second-class electrical component according to the time for restarting; the restarting time of the first-class electrical component is less than a preset time threshold; and the restarting time of the second-class electrical component is greater than or equal to the preset time threshold. The power supply system for the medical X-ray imaging device can effectively reduce the time for reconnecting the medical X-ray imaging device to the power supply to recover to the normal working state.

In this exemplary embodiment, the medical X-ray imaging device is a movable C-shaped arm X-ray machine, and second-class electrical component of the movable C-shaped arm X-ray machine includes an X-ray generator, a computer, a collimator and a micro-control unit; and the X-ray generator belongs to the second-class electrical component needing limitation on operating power in power off.

It is to be understood that, although this description is described according to each embodiment, each embodiment may not include only one independent technical solution. The narrative mode of this description is merely for clarity. This description should be considered as a whole by a person skilled in the art, and the technical solution in each embodiment may also be properly combined, to form other embodiments that can be understood by a person skilled in the art.

The series of detailed explanations listed above are only specific explanations for the feasible embodiments of the present application, and are not intended to limit the scope of protection of the present invention as defined in the attached set of claims.

## Claims

1. A power supply system for a medical X-ray imaging device, comprising:
a power connector (10) configured to be connected to a power grid;
a first power supply connector (21) connected to the power connector (10), the first power supply connector (21) being configured to be connected to a first-class electrical component of the medical X-ray imaging device;
a battery module (30) connected to the power connector (10), the battery module (30) being provided with a storage battery (31), and the battery module (30) being configured to acquire electric energy from the power grid through the power connector (10) to charge the storage battery (31); and
a second power supply connector (22) connected to the battery module (30), the second power supply connector (22) being configured to be connected to a second-class electrical component of the medical X-ray imaging device,
**characterized in that** the battery module (30) comprises a first switch (32) and is configured to connect any one of the power connector (10) and the storage battery (31) to the second power supply connector (22) in a switchable mode, wherein the first switch (32) is controlled by the power connector (10); a contact end of the first switch (32) is connected to the power connector (10), the storage battery (31) and the second power supply connector (22); the first switch (32) is configured to connect the power connector (10) to the second power supply connector (22) in a case that the power connector (10) is in a power-on state; and the first switch (32) is configured to connect the storage battery (31) to the second power supply connector (22) in a case that the power connector (10) is in a power-off state.

2. The power supply system for a medical X-ray imaging device according to claim 1, wherein the battery module (30) comprises:
a first AC/DC converter (33) connected between the power connector (10) and the storage battery (31); and
a first DC/AC converter (34) connected between the storage battery (31) and the first switch (32).

3. The power supply system for a medical X-ray imaging device according to claim 1 or 2, wherein the power supply system further comprises a second AC/DC converter (42); and the second AC/DC converter (42) is connected to the first power supply connector (21) and is configured to be connected to a DC-powered first-class electrical component (82).

4. The power supply system for a medical X-ray imaging device according to claim 1 or 2, wherein the power supply system further comprises a third AC/DC converter (43); and the third AC/DC converter (43) is connected to the second power supply connector (22) and configured to be connected to a DC-powered second-class electrical component (92).

5. The power supply system for a medical X-ray imaging device according to claim 1 or 2, wherein the power supply system comprises two second power supply connectors (22); one second power supply connector (22) is configured to be connected to an AC-powered second-class electrical component, and the other second power supply connector (22) is configured to be connected to a DC-powered second-class electrical component; the contact end of the first switch (32) is provided with a first contact set and a second contact set; the first contact set is connected to the second power supply connector (22) which is configured to be connected to the AC-powered second-class electrical component, and the second contact set is connected to the second power supply connector (22) which is configured to be connected to the DC-powered second-class electrical component; and the battery module (30) further comprises:
a first AC/DC converter (33) connected between the power connector (10) and the storage battery (31);
a first DC/AC converter (34) connected between the storage battery (31) and the first contact set; and
a DC/DC converter (35) connected between the storage battery (31) and the second contact set.

6. The power supply system for a medical X-ray imaging device according to claim 5, wherein the power supply system further comprises a second AC/DC converter (42); the second AC/DC converter (42) is connected to the first power supply connector (21) and configured to be connected to a DC-powered first-class electrical component; and the second contact set is connected to the power connector (10) through the second AC/DC converter (42) and the first power supply connector (21).

7. The power supply system for a medical X-ray imaging device according to any one of claims 1 to 6, wherein the battery module (30) further comprises a battery management system (36); the battery management system (36) is configured to detect a residual electric quantity of the storage battery (31) and transmit an electric quantity signal; the power supply system further comprises a prompt module (50); and the prompt module (50) is connected to the battery management system (36) and is configured to display patterns or make a sound according to the electric quantity signal.

8. The power supply system for a medical X-ray imaging device according to any one of claims 1 to 3, wherein the battery module (30) is an uninterruptible power supply; the power supply system further comprises a second switch (60) and a third power supply connector (23); the third power supply connector (23) is configured to be connected to a second-class electrical component needing limitation on operating power in power off; the second switch (60) is controlled by the power connector (10); a contact end of the second switch (60) is connected to the power connector (10), the second power supply connectors (22) and the third power supply connector (23); and the second switch (60) is configured to connect the power connector (10) to the third power supply connector (23) in a case that the power connector (10) is in the power-on state; and the second switch (60) is configured to connect the second power supply connector (22) to the third power supply connector (23) in a case that the power connector (10) is in the power-off state.

9. The power supply system for a medical X-ray imaging device according to claim 8, wherein the power supply system further comprises a monitoring module (70); the monitoring module is configured to detect an on-off state of the contact end of the second switch (60) and transmit an operating power limiting signal in a case of detecting that the second switch (60) connects the second power supply connectors (22) to the third power supply connector (23); and a control system for a medical X-ray imaging device is configured to control the second-class electrical component needing limitation on operating power in power off to only implement a function that the power is less than a set threshold according to the operating power limiting signal.

10. The power supply system for a medical X-ray imaging device according to any one of claims 1 to 9, wherein the power supply system further comprises an AC/AC converter (44); and the AC/AC converter (44) is connected between the power connector (10) and the first power supply connector (21) and between the power connector (10) and the battery module (30).

11. A medical X-ray imaging device, comprising the power supply system according to any one of claims 1 to 10.

12. The medical X-ray imaging device according to claim 11, wherein the electrical components of the medical X-ray imaging device are divided into the first-class electrical component and the second-class electrical component according to the time for restarting; the restarting time of the first-class electrical component is less than a preset time threshold; and the restarting time of the second-class electrical component is greater than or equal to the preset time threshold.

13. The medical X-ray imaging device according to claim 12, wherein the medical X-ray imaging device is a movable C-shaped arm X-ray machine; and the second-class electrical component of the movable C-shaped arm X-ray machine comprises an X-ray generator, a computer, a collimator and a micro-control unit.

14. The medical X-ray imaging device according to any one of claims 11 to 13, wherein the first-class electrical components comprise an AC-powered first-class electrical component (81) directly connected to the first power supply connector (21).

15. The medical X-ray imaging device according to any one of claims 11 to 14, wherein the second-class electrical components comprise an AC-powered second-class electrical component (91) directly connected to the second power supply connector (22).

## Patentansprüche

1. Stromversorgungssystem für eine medizinische Röntgenbildgebungsvorrichtung, umfassend:
einen Stromanschluss (10), der dazu ausgelegt ist, mit einem Stromnetz verbunden zu werden;
einen ersten Stromversorgungsanschluss (21), der mit dem Stromanschluss (10) verbunden ist, wobei der erste Stromversorgungsanschluss (21) dazu ausgelegt ist, mit einer elektrischen Komponente erster Klasse der medizinischen Röntgenbildgebungsvorrichtung verbunden zu werden;
ein Batteriemodul (30), das mit dem Stromanschluss (10) verbunden ist, wobei das Batteriemodul (30) mit einer Speicherbatterie (31) bereitgestellt wird und das Batteriemodul (30) dazu ausgelegt ist, elektrische Energie über den Stromanschluss (10) aus dem Stromnetz zu beziehen, um die Speicherbatterie (31) zu laden; und
einen zweiten Stromversorgungsanschluss (22), der mit dem Batteriemodul (30) verbunden ist, wobei der zweite Stromversorgungsanschluss (22) dazu ausgelegt ist, mit einer elektrischen Komponente zweiter Klasse der medizinischen Röntgenbildgebungsvorrichtung verbunden zu werden,
**dadurch gekennzeichnet, dass** das Batteriemodul (30) einen ersten Schalter (32) umfasst und dazu ausgelegt ist, eines von dem Stromanschluss (10) und der Speicherbatterie (31) mit dem zweiten Stromversorgungsanschluss (22) in einem umschaltbaren Modus zu verbinden, wobei der erste Schalter (32) durch den Stromanschluss (10) gesteuert wird; ein Kontaktende des ersten Schalters (32) mit dem Stromanschluss (10), der Speicherbatterie (31) und dem zweiten Stromversorgungsanschluss (22) verbunden ist; der erste Schalter (32) dazu ausgelegt ist, den Stromanschluss (10) mit dem zweiten Stromversorgungsanschluss (22) zu verbinden, falls sich der Stromanschluss (10) in einem eingeschalteten Zustand befindet; und der erste Schalter (32) dazu ausgelegt ist, die Speicherbatterie (31) mit dem zweiten Stromversorgungsanschluss (22) zu verbinden, falls sich der Stromanschluss (10) in einem ausgeschalteten Zustand befindet.

2. Stromversorgungssystem für eine medizinische Röntgenbildgebungsvorrichtung gemäß Anspruch 1, wobei das Batteriemodul (30) dies umfasst:
einen ersten AC/DC-Wandler (33), der zwischen dem Stromanschluss (10) und der Speicherbatterie (31) angeschlossen ist; und
einen ersten DC/AC-Wandler (34), der zwischen der Speicherbatterie (31) und dem ersten Schalter (32) angeschlossen ist.

3. Stromversorgungssystem für eine medizinische Röntgenbildgebungsvorrichtung gemäß Anspruch 1 oder 2, wobei das Stromversorgungssystem ferner einen zweiten AC/DC-Wandler (42) umfasst; und der zweite AC/DC-Wandler (42) mit dem ersten Stromversorgungsanschluss (21) verbunden ist und dazu ausgelegt ist, mit einer DC-gespeisten elektrischen Komponente erster Klasse (82) verbunden zu werden.

4. Stromversorgungssystem für eine medizinische Röntgenbildgebungsvorrichtung gemäß Anspruch 1 oder 2, wobei das Stromversorgungssystem ferner einen dritten AC/DC-Wandler (43) umfasst; und der dritte AC/DC-Wandler (43) mit dem zweiten Stromversorgungsanschluss (22) verbunden ist und dazu ausgelegt ist, mit einer DC-gespeisten elektrischen Komponente zweiter Klasse (92) verbunden zu werden.

5. Stromversorgungssystem für eine medizinische Röntgenbildgebungsvorrichtung gemäß Anspruch 1 oder 2, wobei das Stromversorgungssystem zwei zweite Stromversorgungsanschlüsse (22) umfasst; ein zweiter Stromversorgungsanschluss (22) dazu ausgelegt ist, mit einer AC-gespeisten elektrischen Komponente zweiter Klasse verbunden zu werden, und der andere zweite Stromversorgungsanschluss (22) dazu ausgelegt ist, mit einer DC-gespeisten elektrischen Komponente zweiter Klasse verbunden zu werden; das Kontaktende des ersten Schalters (32) mit einem ersten Kontaktsatz und einem zweiten Kontaktsatz bereitgestellt wird; der erste Kontaktsatz mit dem zweiten Stromversorgungsanschluss (22) verbunden ist, der dazu ausgelegt ist, mit der AC-gespeisten elektrischen Komponente zweiter Klasse verbunden zu werden, und der zweite Kontaktsatz mit dem zweiten Stromversorgungsanschluss (22) verbunden ist, der dazu ausgelegt ist, mit der DC-gespeisten elektrischen Komponente zweiter Klasse verbunden zu werden; und das Batteriemodul (30) ferner umfasst:
einen ersten AC/DC-Wandler (33), der zwischen dem Stromanschluss (10) und der Speicherbatterie (31) angeschlossen ist;
einen ersten DC/AC-Wandler (34), der zwischen der Speicherbatterie (31) und dem ersten Kontaktsatz angeschlossen ist; und
einen DC/DC-Wandler (35), der zwischen der Speicherbatterie (31) und dem zweiten Kontaktsatz angeschlossen ist.

6. Stromversorgungssystem für eine medizinische Röntgenbildgebungsvorrichtung gemäß Anspruch 5, wobei das Stromversorgungssystem ferner einen zweiten AC/DC-Wandler (42) umfasst; der zweite AC/DC-Wandler (42) mit dem ersten Stromversorgungsanschluss (21) verbunden ist und dazu ausgelegt ist, mit einer DC-gespeisten elektrischen Komponente erster Klasse verbunden zu werden; und der zweite Kontaktsatz über den zweiten AC/DC-Wandler (42) und den ersten Stromversorgungsanschluss (21) mit dem Stromanschluss (10) verbunden ist.

7. Stromversorgungssystem für eine medizinische Röntgenbildgebungsvorrichtung gemäß einem der Ansprüche 1 bis 6, wobei das Batteriemodul (30) ferner ein Batteriemanagementsystem (36) umfasst; das Batteriemanagementsystem (36) dazu ausgelegt ist, eine elektrische Restmenge der Speicherbatterie (31) zu detektieren und ein elektrisches Mengensignal zu übertragen; das Stromversorgungssystem ferner eine Aufforderungsmodul (50) umfasst; und das Aufforderungsmodul (50) mit dem Batteriemanagementsystem (36) verbunden ist und dazu ausgelegt ist, entsprechend dem elektrischen Mengensignal Muster anzuzeigen oder ein Geräusch zu erzeugen.

8. Stromversorgungssystem für eine medizinische Röntgenbildgebungsvorrichtung gemäß einem der Ansprüche 1 bis 3, wobei das Batteriemodul (30) eine unterbrechungsfreie Stromversorgung ist; das Stromversorgungssystem ferner einen zweiten Schalter (60) und einen dritten Stromversorgungsanschluss (23) umfasst; der dritte Stromversorgungsanschluss (23) dazu ausgelegt ist, mit einer elektrischen Komponente zweiter Klasse verbunden zu werden, die eine Begrenzung der Betriebsleistung im ausgeschalteten Zustand erfordert; der zweite Schalter (60) durch den Stromanschluss (10) gesteuert wird; ein Kontaktende des zweiten Schalters (60) mit dem Stromanschluss (10), den zweiten Stromversorgungsanschlüssen (22) und dem dritten Stromversorgungsanschluss (23) verbunden ist; und der zweite Schalter (60) dazu ausgelegt ist, den Stromanschluss (10) mit dem dritten Stromversorgungsanschluss (23)zu verbinden, falls sich der Stromanschluss (10) im eingeschalteten Zustand befindet; und der zweite Schalter (60) dazu ausgelegt ist, den zweiten Stromversorgungsanschluss (22) mit dem dritten Stromversorgungsanschluss (23) zu verbinden, falls sich der Stromanschluss (10) im ausgeschalteten Zustand befindet.

9. Stromversorgungssystem für eine medizinische Röntgenbildgebungsvorrichtung gemäß Anspruch 8, wobei das Stromversorgungssystem ferner ein Überwachungsmodul (70) umfasst; das Überwachungsmodul dazu ausgelegt ist, einen Ein-Aus-Zustand des Kontaktendes des zweiten Schalters (60) zu detektieren und ein Betriebsleistungsbegrenzungssignal zu übertragen, falls detektiert wird, dass der zweite Schalter (60) die zweiten Stromversorgungsanschlüsse (22) mit dem dritten Stromversorgungsanschluss (23) verbindet; und ein Steuerungssystem für eine medizinische Röntgenbildgebungsvorrichtung dazu ausgelegt ist, die elektrische Komponente zweiter Klasse, welche eine Begrenzung der Betriebsleistung im ausgeschalteten Zustand erfordert, zu steuern, um nur eine Funktion zu implementieren, dass die Leistung geringer als ein eingestellter Schwellwert gemäß dem Betriebsleistungsbegrenzungssignal ist.

10. Stromversorgungssystem für eine medizinische Röntgenbildgebungsvorrichtung gemäß einem der Ansprüche 1 bis 9, wobei das Stromversorgungssystem ferner einen AC/AC-Wandler (44) umfasst; und der AC/AC-Wandler (44) zwischen dem Stromanschluss (10) und dem ersten Stromversorgungsanschluss (21) und zwischen dem Stromanschluss (10) und dem Batteriemodul (30) angeschlossen ist.

11. Medizinische Röntgenbildgebungsvorrichtung, umfassend das Stromversorgungssystem gemäß einem der Ansprüche 1 bis 10.

12. Medizinische Röntgenbildgebungsvorrichtung gemäß Anspruch 11, wobei die elektrischen Komponenten der medizinischen Röntgenbildgebungsvorrichtung entsprechend der Zeit für einen Neustart in die elektrische Komponente erster Klasse und die elektrische Komponente zweiter Klasse unterteilt sind; die Neustartzeit der elektrischen Komponente erster Klasse ist kleiner als ein voreingestellter Zeitschwellwert; und die Neustartzeit der elektrischen Komponente zweiter Klasse ist größer oder gleich dem voreingestellten Zeitschwellwert.

13. Medizinische Röntgenbildgebungsvorrichtung gemäß Anspruch 12, wobei die medizinische Röntgenbildgebungsvorrichtung eine Röntgenmaschine mit beweglichem C-förmigen Arm ist; und die elektrische Komponente zweiter Klasse der Röntgenmaschine mit beweglichem C-förmigen Arm einen Röntgengenerator, einen Computer, einen Kollimator und eine Mikrosteuerungseinheit umfasst.

14. Medizinische Röntgenbildgebungsvorrichtung gemäß einem der Ansprüche 11 bis 13, wobei die elektrischen Komponenten erster Klasse eine AC-gespeiste elektrische Komponente erster Klasse (81) umfassen, die direkt mit dem ersten Stromversorgungsanschluss (21) verbunden ist.

15. Medizinische Röntgenbildgebungsvorrichtung gemäß einem der Ansprüche 11 bis 14, wobei die elektrischen Komponenten zweiter Klasse eine AC-gespeiste elektrische Komponente zweiter Klasse (91) umfassen, die direkt mit dem zweiten Stromversorgungsanschluss (22) verbunden ist.

## Revendications

1. Système d'alimentation électrique pour un dispositif d'imagerie médicale à rayons X, comprenant :
un connecteur d'alimentation (10) configuré pour être connecté à un réseau électrique ;
un premier connecteur d'alimentation électrique (21) connecté au connecteur d'alimentation (10), le premier connecteur d'alimentation électrique (21) étant configuré pour être connecté à un composant électrique de première classe du dispositif d'imagerie médicale à rayons X ;
un module de batterie (30) connecté au connecteur électrique (10), le module de batterie (30) étant pourvu d'une batterie de stockage (31), et le module de batterie (30) étant configuré pour acquérir de l'énergie électrique à partir du réseau électrique par le biais du connecteur électrique (10) pour charger la batterie de stockage (31) ; et
un deuxième connecteur d'alimentation électrique (22) connecté au module de batterie (30), le deuxième connecteur d'alimentation électrique (22) étant configuré pour être connecté à un composant électrique de seconde classe du dispositif d'imagerie médicale à rayons X,
**caractérisé en ce que** le module de batterie (30) comprend un premier commutateur (32) et est configuré pour connecter l'un quelconque du connecteur électrique (10) et de la batterie de stockage (31) au deuxième connecteur d'alimentation électrique (22) dans un mode commutable, dans lequel le premier commutateur (32) est commandé par le connecteur électrique (10) ; une extrémité de contact du premier commutateur (32) est connectée au connecteur électrique (10), à la batterie de stockage (31) et au deuxième connecteur d'alimentation électrique (22) ; le premier commutateur (32) est configuré pour connecter le connecteur électrique (10) au deuxième connecteur d'alimentation électrique (22) dans le cas où le connecteur électrique (10) est dans un état de mise sous tension ; et le premier commutateur (32) est configuré pour connecter la batterie de stockage (31) au deuxième connecteur d'alimentation électrique (22) dans le cas où le connecteur électrique (10) est dans un état de mise hors tension.

2. Système d'alimentation électrique pour un dispositif médical d'imagerie à rayons X selon la revendication 1, dans lequel le module de batterie (30) comprend :
un premier convertisseur CA/CC (33) connecté entre le connecteur électrique (10) et la batterie de stockage (31) ; et
un premier convertisseur CC/CA (34) connecté entre la batterie de stockage (31) et le premier commutateur (32).

3. Système d'alimentation électrique pour un dispositif d'imagerie à rayons X médicale selon la revendication 1 ou 2, dans lequel le système d'alimentation électrique comprend en outre un deuxième convertisseur CA/CC (42) ; et le deuxième convertisseur CA/CC (42) est connecté au premier connecteur d'alimentation électrique (21) et est configuré pour être connecté à un composant électrique de première classe alimenté en courant continu (82).

4. Système d'alimentation électrique pour un dispositif d'imagerie à rayons X médicale selon la revendication 1 ou 2, dans lequel le système d'alimentation électrique comprend en outre un troisième convertisseur CA/CC (43) ; et le troisième convertisseur CA/CC (43) est connecté au deuxième connecteur d'alimentation électrique (22) et configuré pour être connecté à un composant électrique de seconde classe alimenté en courant continu (92).

5. Système d'alimentation électrique pour un dispositif d'imagerie médicale à rayons X selon la revendication 1 ou 2, dans lequel le système d'alimentation électrique comprend deux seconds connecteurs d'alimentation électrique (22) ; un deuxième connecteur d'alimentation électrique (22) est configuré pour être connecté à un composant électrique de seconde classe alimenté en courant alternatif, et l'autre deuxième connecteur d'alimentation électrique (22) est configuré pour être connecté à un composant électrique de seconde classe alimenté en courant continu ; l'extrémité de contact du premier commutateur (32) est pourvue d'un premier ensemble de contacts et d'un second ensemble de contacts ; le premier ensemble de contacts est connecté au deuxième connecteur d'alimentation électrique (22) qui est configuré pour être connecté au composant électrique de seconde classe alimenté en courant alternatif, et le second ensemble de contacts est connecté au deuxième connecteur d'alimentation électrique (22) qui est configuré pour être connecté au composant électrique de seconde classe alimenté en courant continu ; et le module de batterie (30) comprend en outre :
un premier convertisseur CA/CC (33) connecté entre le connecteur électrique (10) et la batterie de stockage (31) ;
un premier convertisseur CC/CA (34) connecté entre la batterie de stockage (31) et le premier ensemble de contacts ; et
un convertisseur CC/CC (35) connecté entre la batterie de stockage (31) et le second ensemble de contacts.

6. Système d'alimentation électrique pour un dispositif d'imagerie médicale à rayons X selon la revendication 5, dans lequel le système d'alimentation électrique comprend en outre un deuxième convertisseur CA/CC (42) ; le deuxième convertisseur CA/CC (42) est connecté au premier connecteur d'alimentation électrique (21) et configuré pour être connecté à un composant électrique de première classe alimenté en courant continu ; et le second ensemble de contacts est connecté au connecteur d'alimentation (10) par l'intermédiaire du deuxième convertisseur CA/CC (42) et du premier connecteur d'alimentation électrique (21).

7. Système d'alimentation électrique pour un dispositif d'imagerie médicale à rayons X selon l'une quelconque des revendications 1 à 6, dans lequel le module de batterie (30) comprend en outre un système de gestion de batterie (36) ; le système de gestion de batterie (36) est configuré pour détecter une quantité électrique résiduelle de la batterie de stockage (31) et transmettre un signal de quantité électrique ; le système d'alimentation électrique comprend en outre un module d'invite (50) ; et le module d'invite (50) est connecté au système de gestion de batterie (36) et est configuré pour afficher des motifs ou émettre un son selon le signal de quantité électrique.

8. Système d'alimentation électrique pour un dispositif d'imagerie médicale à rayons X selon l'une quelconque des revendications 1 à 3, dans lequel le module de batterie (30) est une alimentation électrique sans interruption ; le système d'alimentation électrique comprend en outre un second commutateur (60) et un troisième connecteur d'alimentation électrique (23) ; le troisième connecteur d'alimentation électrique (23) est configuré pour être connecté à un composant électrique de seconde classe nécessitant une limitation de la puissance de fonctionnement hors tension ; le second commutateur (60) est commandé par le connecteur d'alimentation électrique (10) ; une extrémité de contact du second commutateur (60) est connectée au connecteur électrique (10), aux deuxièmes connecteurs d'alimentation électrique (22) et au troisième connecteur d'alimentation (23) ; et le second commutateur (60) est configuré pour connecter le connecteur électrique (10) au troisième connecteur d'alimentation (23) dans le cas où le connecteur électrique (10) est dans l'état de mise sous tension ; et le second commutateur (60) est configuré pour connecter le deuxième connecteur d'alimentation électrique (22) au troisième connecteur d'alimentation (23) dans le cas où le connecteur électrique (10) est dans l'état de mise hors tension.

9. Système d'alimentation électrique pour un dispositif d'imagerie médicale à rayons X selon la revendication 8, dans lequel le système d'alimentation électrique comprend en outre un module de surveillance (70) ; le module de surveillance est configuré pour détecter un état sous tension/hors tension de l'extrémité de contact du second commutateur (60) et transmettre un signal de limitation de puissance de fonctionnement dans un cas où il est détecté que le second commutateur (60) connecte les deuxièmes connecteurs d'alimentation électrique (22) au troisième connecteur d'alimentation électrique (23) ; et un système de commande pour un dispositif d'imagerie médicale à rayons X est configuré pour commander le composant électrique de seconde classe nécessitant une limitation de la puissance de fonctionnement en mode hors tension pour ne mettre en œuvre qu'une fonction selon laquelle la puissance est inférieure à un seuil défini en fonction du signal de limitation de puissance de fonctionnement.

10. Système d'alimentation électrique pour un dispositif d'imagerie médicale à rayons X selon l'une quelconque des revendications 1 à 9, dans lequel le système d'alimentation électrique comprend en outre un convertisseur CA/CA (44) ; et le convertisseur CA/CA (44) est connecté entre le connecteur d'alimentation (10) et le premier connecteur d'alimentation électrique (21) et entre le connecteur d'alimentation (10) et le module de batterie (30).

11. Dispositif d'imagerie médicale à rayons X, comprenant le système d'alimentation électrique selon l'une quelconque des revendications 1 à 10.

12. Dispositif d'imagerie médicale à rayons X selon la revendication 11, dans lequel les composants électriques du dispositif d'imagerie médicale à rayons X sont divisés en composants électriques de première classe et composants électriques de seconde classe selon le temps de redémarrage ; le temps de redémarrage du composant électrique de première classe est inférieur à un seuil de temps prédéfini ; et le temps de redémarrage du composant électrique de seconde classe est supérieur ou égal au seuil de temps prédéfini.

13. Dispositif d'imagerie médicale à rayons X selon la revendication 12, dans lequel le dispositif d'imagerie médicale à rayons X est une machine à rayons X à bras mobile en forme de C ; et le composant électrique de seconde classe de la machine à rayons X à bras mobile en forme de C comprend un générateur de rayons X, un ordinateur, un collimateur et une unité de microcontrôle.

14. Dispositif d'imagerie médicale à rayons X selon l'une quelconque des revendications 11 à 13, dans lequel les composants électriques de première classe comprennent un composant électrique de première classe alimenté en courant alternatif (81) directement connecté au premier connecteur d'alimentation électrique (21).

15. Dispositif d'imagerie médicale à rayons X selon l'une quelconque des revendications 11 à 14, dans lequel les composants électriques de seconde classe comprennent un composant électrique de seconde classe alimenté en courant alternatif (91) directement connecté au second connecteur d'alimentation électrique (22).
